(19) Europäisches Patentamt
European Patent Office
Office européen des brevets

(11) Publication number: 0 318 162
A2

(12) # EUROPEAN PATENT APPLICATION

(21) Application number: 88310185.9

(51) Int. Cl.4: C07D 207/452 , C08G 73/12

(22) Date of filing: 28.10.88

(30) Priority: 25.11.87 US 125086
25.11.87 US 125093

(43) Date of publication of application:
31.05.89 Bulletin 89/22

(84) Designated Contracting States:
FR GB NL SE

(71) Applicant: TEXACO DEVELOPMENT CORPORATION
2000 Westchester Avenue
White Plains New York 10650(US)

(72) Inventor: Alexander, David Christopher
4900 Black Angus Cove
Austin Texas 78759(US)
Inventor: Speranza, George Phillip
2800 Silverleaf Circle
Austin Texas 78757(US)

(74) Representative: Harrison, Michael Robert et al
Urquhart-Dykes & Lord 91 Wimpole Street
London W1M 8AH(GB)

(54) **Novel bismaleimide derivatives.**

(57) Novel bismaleimides of the formula

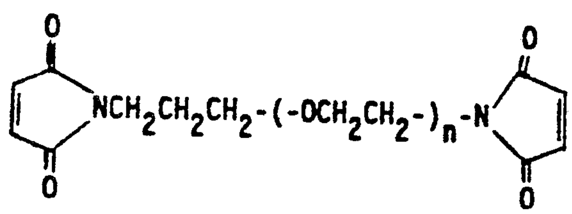

wherein n = 1,2 or 3 are formed in two steps by reacting a diamine and maleic anhydride to form a bismaleic acid which is reacted with acetic anhydride in the presence of acetone to form the bismaleimide. This can then be further reacted with a high molecular weight polyoxyalkyleneamine resulting in a novel bismaleimide derivative of a higher molecular weight polyoxyalkyleneamine, which derivative has the general formula:

EP 0 318 162 A2

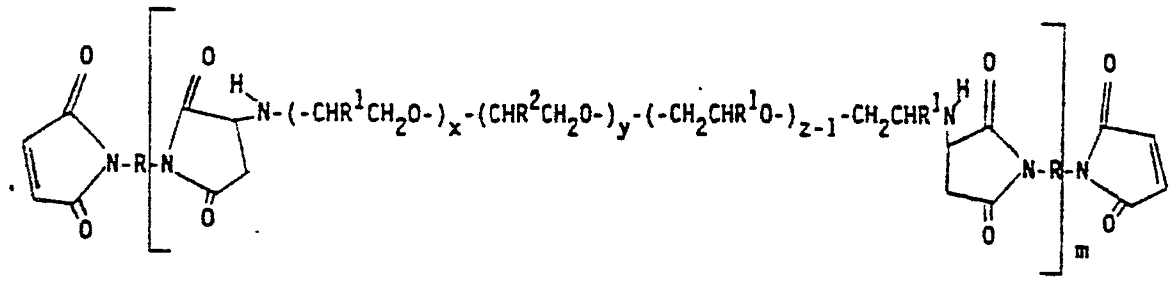

$R=CH_2CH_2-(OCH_2CH_2-)_n-$

wherein $R^1$ and $R^2$ are independently alkyl or hydrogen, x, y and z depend on the amine used, n is 1, 2 or 3 and m is predominantly 1.

# NOVEL BISMALEIMIDE DERIVATIVES

This invention relates to bismaleimides. More particularly, this invention relates to novel bismaleimide derivatives obtained from oxyethylene diamines and anhydrides or higher molecular weight polyoxyalkylene amines.

Bismaleimide polymers are important primarily for their thermal stability, which usually results from aromaticity in the structure. Although the majority of bismaleimides have therefore been prepared from aromatic diamines, aliphatic compounds can also provide benefits in improved processibility, flexibility and solubility. Bismaleimides have, for example, been prepared from alkylene-diamines (J. Appl. Poly. Sci., 29, 891-899 (1984) and from JEFFAMINE® ED-diamines (U.S. Patent 3,951,502). In a series of Japanese patents issued to Mitsui Toatsu Chemicals (JP 82 205,413; 83 40,374; 83 15,515; 83 136,637), bismaleimides were also prepared from diamines such as 4,7-dioxadecane-1,10-diamine (reduction product of cyanoethylated ethylene glycol) and related diamines; these are used with polybutadiene in preparation of copolymers. The maleimide of triethylene glycol monoamine is also reported in one of these patents. Use of the oxyethylene group to increase flexibility has been effective in some other types of polymers as well. In J. Macromol. Sci.--Chem., A21, 1117-1135 (1984) there is described preparation of "reactive plasticizers" with acetylene endgroups and internal oxyethylene groups. Stenzenberger, in German Patent No. 2,127,024 disclosed the preparation of an aliphatic bismaleimide from 2,2,4-trimethylhexane-1,6-diamine and in German Patent No. 2,165,974 he described its thermal polymerization.

The use of mixtures of polyoxyalkylene bismaleimides (with molecular weights greater than 400) and aromatic bismaleimides in preparation of flexibilized polybismaleimides is disclosed by de Koning in European Patent Application No. 206,383. While the heat distortion temperature fell with increasing amounts of flexibilizing bismaleimide, the elongation and flexure at break both increased.

In U. S. Patent No. 4,237,262, Jones discloses a low temperature curable composition comprising at least one curable polyimide prepolymer formed by heating an aliphatic oxyalkylene bismaleimide with an aromatic polyamine and at least one aromatic bismaleimide and at least one aliphatic epoxy resin. The reaction product provides at least two functional epoxy groups to provide a low temperature curable composition. In U.S. Patent No. 3,951,902 Jones et al. disclose a compliant polyimide having superior thermal mechanical properties produced by reacting an aromatic bis(furfurylimide) with an aliphatic ether bis(maleimide) via a Diels-Alder reaction.

In U. S. Patent No. 4,116,937, Jones also discloses a resin system prepared by Michael addition of a mixture of oxyalkylene and aromatic bismaleimides to aromatic diamines. The oxyalkylene bismaleimides have molecular weights of about 750, and the product is a glassy solid at room temperature.

In the work described in 4,116,937 the objective was to make elastomers. The elongations for the polymers described in the examples therein range from 70% to 170%. These polymers were probably not very rigid, this property being a function of the molecular weight of the amines used and the distance between maleimide units. Another disadvantage is that the amines used here are aromatic amines, which are in many cases known or suspected to be carcinogenic or otherwise toxic; although the issue is not specifically addressed, it is not likely the "flexible polyimide precursor", with its aromatic content, would be soluble to any great extent in water.

Nagasaki, in European Patent Application 191,931, reveals the use of certain oxyalkylene bismaleimides in rubber compositions.

A curable resin composition is disclosed in Jpn. Kokai Tokkyo Koho JP 58, 136,637 [83,136,637] 13 Aug. 1983 to Mitsui Toatsu Chemicals. The compounds contains an aliphatic imide and polybutadiene containing double bonds.

A Japanese Patent to Mitsui Toatsu Chemicals, Inc. (JP 58,127,735 [83,127,735] (Cl. CO8G 73/10), 29 Jul. 1983) discloses heat resistant electrical insulators for printed circuit boards which are prepared from mixtures of aliphatic polyether bisimides, aromatic bisimides and diamines.

An article by White in Ind. Eng. Chem. Prod. Res. Dev. 25, 395-400 discusses the fact that bisimides offer potential for the synthesis of high-molecular-weight, step growth polymers. It is stated they are flanked by two electron-withdrawing carbonyl groups, and the electrophilic maleimide carbon-carbon-double bond is especially labile to nucleophilic attack and yields Michael type adducts with both amines and thiols. The paper focuses on the requirements for preparation of these polymeric Michael adducts, with additional emphasis on the effects of the enormous structural variety available within the class in the thermal and physical properties of these new resins.

In the art experimental data are available wherein polymers were synthesized which are structurally

related to those formed by nucleophilic or Michael addition of diaminoarenes, but which had more flexible backbones and lower glass transition temperature (Tg). See "Reaction of Diaminoalkanes with Bismaleimides: Synthesis of Some Unusual Polyimides", Journal of Applied Polymer Science, Vol. 29, 891-899 (1984).

Shaw and Kinloch have studied the effects of rubber concentration on the morphology, bulk mechanical and thermal properties and the adhesive strength of the bismaleimide by the addition of various amounts of a carbonyl-terminated butadiene (CTBN) rubber toughening agent, and concluded that surprisingly large amounts of CTBN rubber can be added to substantially improve the fracture resistance of the bismaleimide resin without sacrificing other important properties. (see "Toughened Bismaleimide Adhesives", Int.J Adhesion, July 1985, pp. 123-127.)

A growing number of applications for polyimides are discussed in an article titled "Premium Performance from Polyimides" in ME, January 1986, p. 14-19.

In U.S. Patent No. 4,277,582 Mueller discloses water-insoluble hydrophilic copolymers consisting of a hydrophilic polymer of monoolefinic monomers cross-linked with a major amount of a diolefinic non-hydrophilic macromer.

Polymerization of the bismaleimide of dimer diamine, which also contains a hydrocarbon backbone, is disclosed in U.S. Patent No. 4,564,663. The product polymer is hard and thermally stable.

It appears there is a large market for bismaleimides and a good deal of research in the art has been directed toward studying properties of and better methods for producing these compounds. Bismaleimide compounds are increasingly important in high performance polymers commonly used as matrix resin for composites. It is believed that the polybismaleimide derivatives of the instant invention, particularly those derived from di-, tri- and tetraethylene glycol diamines including bisaminoethyl ether, JEFFAMINE® EDR-148 and JEFFAMINE® EDR-192 would be useful as monomers in homopolymers and copolymers and would exhibit advantages including improved flexibility, processibility, toughness and solubility. They could be used alone or in combination with other materials such as aliphatic or aromatic diamines or unsaturated compounds (including aromatic bismaleimides) to give copolymers. Additionally, the polybismaleimide derivatives from higher weight polyoxyalkylene amines have the advantage that aromatic amines, which are in many cases known or suspected to be carcinogenic or otherwise toxic, are not used to prepare the materials.

In another embodiment of the invention monomers are prepared from higher weight polyoxyalkyleneamines having a polyoxyalkylene backbone and aromatic bismaleimide termination.

The products resulting from the latter embodiment would be of particular interest because they are formed from the reaction of two commercially available materials. The method of preparation disclosed is much easier than the direct route, using maleic anhydride. These materials are useful in the preparation of homopolymers and copolymers that may be suitable as matrix resins in composites.

The series of oxyethylene diamines consisting of BAEE (bisaminoethyl ether, or diethylene glycol diamine), JEFFAMINE® EDR-148 (triethylene glycol diamine), and JEFFAMINE® EDR-192 (tetraethylene glycol diamine) are promising candidates for conversion to bismaleimides (eqs. 1,2). Incorporation of bismaleimides made from these diamines could give new prepolymers and polymers with enhanced flexibility, processibility or solubility. The polymer prepared from the adduct of EDR-148 and EDR-148 BMI was a relatively hard material with a high flexural modulus (close to 500,000 psi). In the instant invention there was less distance between the maleimide units than in, for example, the case of 4,116,937, where the amines had a minimum molecular weight of 600, and therefore the instant polymers exhibited more rigidity. Another advantage of the instant invention is that the prepolymer products exhibit solubility in water, unlike products resulting from the use of aromatic amines.

One derivative, in particular, is the bismaleimide of triethylene glycol diamine. This bismaleimide should be quite useful as a monomer, but it is solid. This detracts from the usefulness for many applications. By a method disclosed in another embodiment of the instant invention it is converted to a liquid form suitable for polymerization. Water-soluble bismaleimides and polybismaleimides have been sought in recent years, and compounds such as the prepolymers of this invention could prove to be especially useful in this respect.

In accordance with the present invention there is provided a novel bismaleimide derivative which is of the formula A or B and is formed at a temperature sufficient to cause thermal reaction between heated components of the mixture of:

(a) at least one diamine of the formula

$N_2NCH_2CH_2\text{-}(\text{-}OCH_2CH_2\text{-})_n\text{-}NH_2$

wherein n equals 2, 3 or 4 and

(b) maleic anhydride

wherein components (a) and (b) form a bismaleamic acid represented by the formula

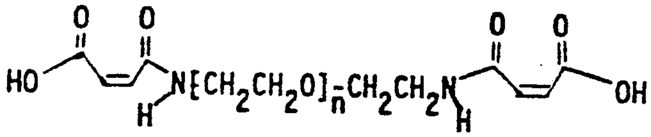

which is further reacted with acetic anhydride to form a bismaleimide of the formula A:

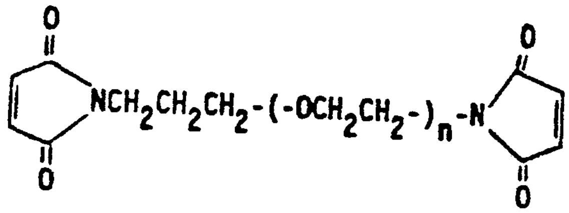

wherein n = 2, 3 or 4, which optionally can be further reacted with a polyoxyalkyleneamine with a molecular weight greater than about 200 resulting in a bismaleimide derivative of a higher molecular weight polyoxyalkyleneamine of formula B:

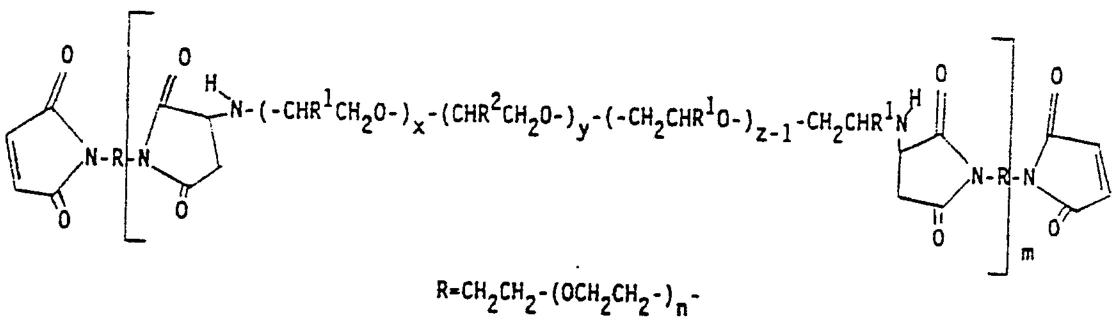

$R=CH_2CH_2-(OCH_2CH_2-)_n-$

wherein $R^1$ and $R^2$ are independently alkyl or hydrogen, x, y and z depend on the amine used, n is 1, 2, or 3, and m is predominantly 1.

The oxyethylene bismaleimide derivatives are prepared from an oxyethylene diamine having the formula:

$H_2NCH_2CH_2-(-OCH_2CH_2-)_n-NH_2$ (I)

where n = 2, 3 or 4 which is combined with maleic anhydride of the formula:

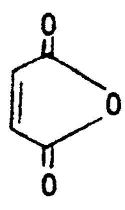

(II)

to form a bismaleamic acid of the formula:

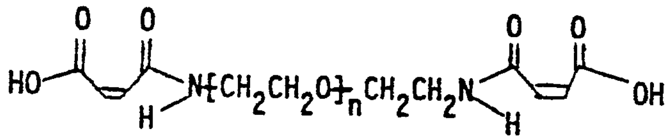

(III)

which is reacted with acetic anhydride in the presence of acetone solution to form a bismaleimide of the formula:

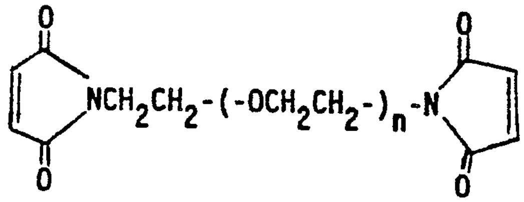

(IV)

where n = 2, 3 or 4.

The novel bismaleimide derivatives of higher molecular weight polyoxyalkyleneamines represented by the formula:

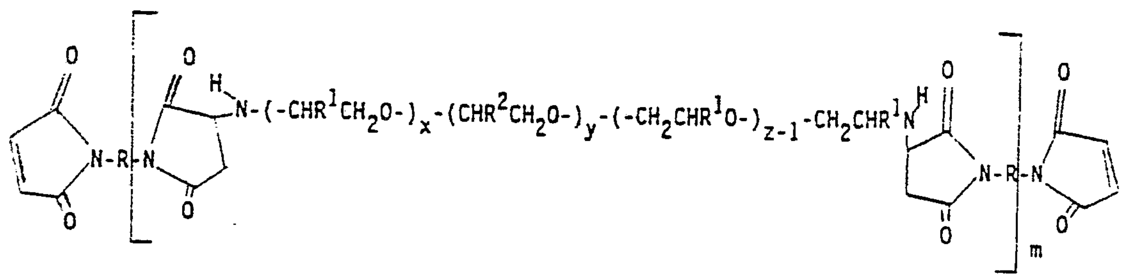

$R{=}CH_2CH_2\text{-}(OCH_2CH_2\text{-})_n\text{-}$

are formed by combining I and II to form III and IV which is then further reacted with a polyoxyalkyleneamine with a molecular weight greater than about 200 to form the above.

In another embodiment aromatic bismaleimide derivatives are prepared by reaction of polyoxyalkyleneamines with commercially availabl;e aromatic bismaleimides. These materials are useful in the preparation of homopolymers and copolymers that may be suitable as matrix resins in composites. This can be represented by the following:

$$H_2N\text{-}(\text{-}CHR^1CH_2O\text{-})_x\text{-}(\text{-}CHR^2CH_2O\text{-})_y\text{-}(\text{-}CH_2CHR^1O\text{-})_{z-1}\text{-}CH_2CHR^1NH_2\ +$$

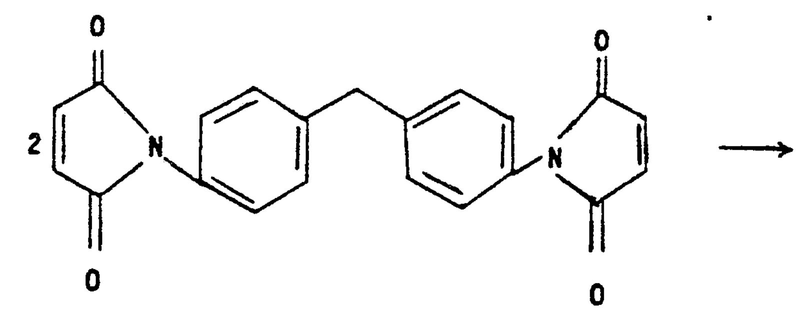

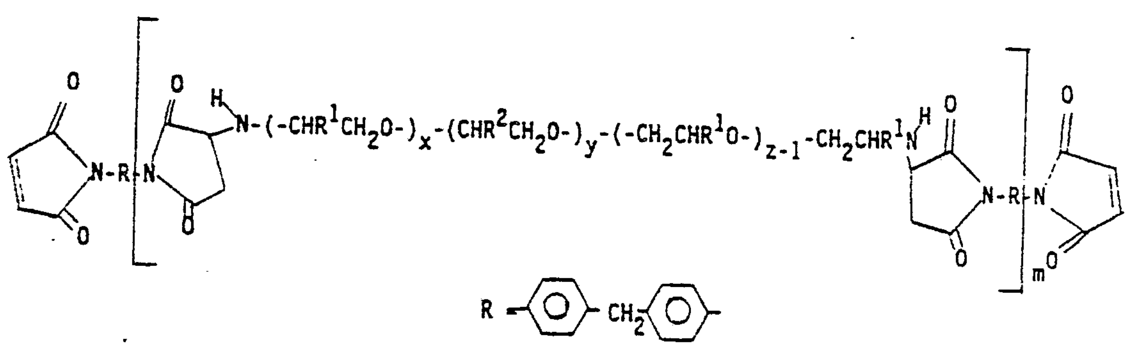

where $R^1$ and $R^2$ are independently alkyl or hydrogen, x, y, and z depend on the amine used, n is 1, 2, or 3, and m is predominantly 1.

Preparation of monomers containing polyoxyalkylene backbones and aromatic bismaleimide termination in this second embodiment is much easier than by direct route (reaction of the polyoxyalkyleneamine with maleic anhydride); from monomers bismaleimide copolymers and homopolymers may be prepared by several methods.

Diamine Starting Materials

The oxyethylene diamine reactants represented by (1) above include di-, tri-, and tetraethylene glycol diamine compounds.

Of special note are the "JEFFAMINE"® EDR series diamines". The structure of "JEFFAMINE® EDR" can be generically illustrated as follows:

$H_2N\{CH_2CH_2O\}_nCH_2CH_2NH_2$

where n = 2, 3.

In one example the diamine used is JEFFAMINE® EDR-148. JEFFAMINE® EDR-148 is the trademark for a triethylene glycol diamine produced by Texaco Inc. In another example the diamine is JEFFAMINE® EDR-192. JEFFAMINE® EDR-192 is the trademark for tetraethylene glycol diamine produced by Texaco.

Preparation of the bismaleimides is effected by reacting a diamine with 2 moles of maleic anhydride. These reactants are mixed with a small amount of solvent to facilitate the mixing process and the solvent is subsequently driven off after the bismaleimide is formed.

Preparation of the Bismaleimides

The bismaleimide product is preferentially formed when a bis(amic acid) (prepared from the oxyethylene diamines and maleic anhydride) is reacted with an excess of anhydride at autogenous pressure at a temperature within the range of about 50°C to about 150°C for a reaction time within the range of about 0.5 to about 12 hours. Good results are obtained by heating the mixture at 60° to 100°C for 0.5 to 4 hours to provide complete reaction of the diamine and the anhydride. Normally, the reaction will to to completion after a reaction time within the range of about 1-2 hours.

The reaction is complete when essentially all of the diamine has reacted with the maleic anhydride. Under the noncatalytic reaction conditions employed herein, the amine groups of the polyoxyalkylene diamine are essentially unreactive with each other.

The bismaleimide monomers and prepolymers that are formed by the process of the present invention are liquid or crystalline solid materials having a molecular weight within the range of about 250 to about 1000 or to about 6000 in the case of the higher molecular weight derivatives containing no terminal primary amine groups.

The reaction mixture will comprise a diamine addition product which may be generally characterised by the following formula:

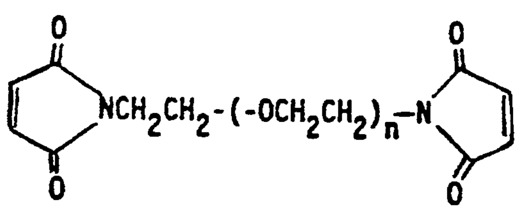

wherein n represents 1, 2, 3 or 4.

A variety of molecular configurations is possible for the bismaleimides of the present invention, depending on the starting materials. For example, where the starting materials are bisaminoethyl ether and maleic anhydride, the bismaleimide will have the formula:

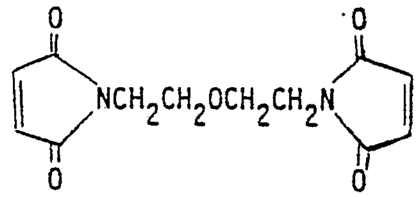

The bismaleimide derivatives of higher molecular weight polyoxyalkyleneamines are formed by reacting the bismaleimide formed by the process described above with high molecular weight polyoxyalkylene amines described below.

Where JEFFAMINE® EDR-192 is reacted with maleic anhydride, the reaction product that is formed will be composed principally of a bismaleimide addition product having the formula:

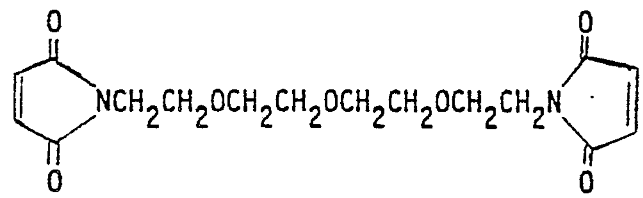

In another embodiment of this invention the solid bismaleimides of this invention are converted to liquid prepolymers.

The significance of this invention is that the bismaleimide of triethylene glycol diamine or other solid oxyethylene bismaleimide should be quite useful as a monomer except that it is a solid, which detracts from its utility for many applications. By conversion into the prepolymer the oxyethylene bismaleimide is unexpectedly converted into a liquid form suitable for polymerization. The liquid products can be cured thermally to give hard polymers. The polymers are potentially useful as matrix resins or components thereof containing ether linkages that may impart improved processibility.

The liquid form is obtained from the bismaleimide by reacting the solid with EDR-148 to give a liquid bismaleimidoaspartimide prepolymer. In many applications liquids are preferable to solids and the liquid prepolymers can be cured thermally.

This can be represented by equation 3:

$H_2NCH_2CH_2OCH_2CH_2OCH_2CH_2NH_2$ +

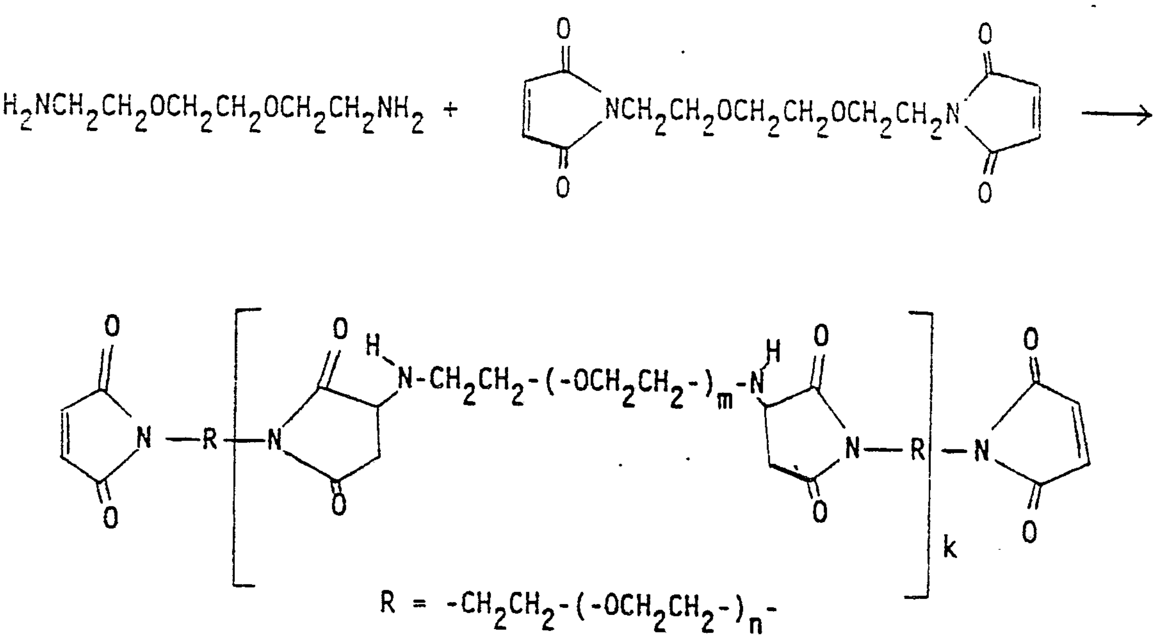

$R = -CH_2CH_2-(-OCH_2CH_2-)_n-$

where m = 2, n = 2 and k is predominantly 1.

Examples For The Preparation of Diamine Bismaleimides From Oxyethylene and Anhydride

The preparation of the prepolymer is carried out at temperatures up to 125° C and polymerization is carried out at subatmospheric or superatmospheric pressures.

In Specific Examples I to VI the preparations of three bismaleamic acids were quite similar and were based on the preparations of bismaleimides from alkylene-diamines reported by White (J. Appl. Poly. Sci 29, 891-899 (1984), incorporated herein by reference.

Specific Example VII demonstrates the preparation of the bismaleimidoaspartimide.

Specific Example VIII demonstrates the use of the bismaleimidoaspartimide in polymer preparation.

The following examples are given in the way of illustration only and are not intended as limitations on the scope of the invention.

## SPECIFIC EXAMPLE I

In the preparation of the bismaleamic acid from triethylene glycol diamine, maleic anhydride (62 g, 630 mmol) was dissolved in 250 mlchloroform; the solution was cooled to 8° C in 500 ml,3-necked round-bottomed flask equipped with magnetic stirrer and nitrogen inlet. Triethylene glycol diamine (48 g, 320 mmol) was added dropwise (with the temperature kept below about 10° C) over a period of three hours, which led to precipitation of a white solid within the first hour. After the addition was complete, the slurry was allowed to warm to room temperature and stirred for two hours more. The mixture was then filtered: after it was dried under vacuum a white powder (105 g, 95%) was obtained, m. p. 150-151.5° C. Titration of this solid showed an acid content of 5.77 meq/g; theoretical acid content is 5.78 meq/g. Spectra (nmr, ir) were consistent with the assigned structure.

## SPECIFIC EXAMPLE II

Preparation of the tetraethylene glycol diamine maleamic acid was carried out similarly, but this material did not precipitate as a solid. Near the end of the addition two phases appeared; the upper phase was a chloroform solution of the bismaleamic acid. After removal of the chloroform the residual oil solidified and gave, after drying under vacuum, a white powder (53 g, 87%), m. p. 96-101° C. Titration of this solid gave an acid content of 5.24 meg/g; theoretical acid content is 5.15 meg/g. Spectra (nmr, ir) were consistent with the assigned structure.

## SPECIFIC EXAMPLE III

The bismaleamic acid from diethylene glycol diamine precipitated immediately on addition of the amine to the anhydride solution and was isolated as a white powder. After it was dried under vacuum 60.3 g (96%) was obtained, m. p. 164-166° C. The acid content was found by titration to be 6.73 meq/g; the theoretical acid content is 6.67 meq/g. Spectra (nmr, ir) were consistent with the assigned structure.

## SPECIFIC EXAMPLES IV-VI

Examples IV through VI represent the second step of the two-step process wherein the bismaleimides are prepared from the bismaleamic acids.

The bismaleimides were prepared by reaction of the bismaleamic acid with acetic anhydride in acetone solution in the presence of triethylamine and acetate salts.

## SPECIFIC EXAMPLE IV

In the preparation of triethylene glycol diamine bismaleimide, the bismaleamic acid (60 g, 0.17 mol), triethylamine (11.5 g, 0.11 mol), and sodium acetate trihydrate (1.9 g, 0.13 mol)) were added to acetone (200 ml) in a 1000 ml 3-necked round bottomed flask fitted with nitrogen inlet and magnetic stirrer. Acetic anhydride (110 g, 1.08 mol) was added, and the resulting mixture was heated at reflux (70°) for 2.5 hours; during this period the solid dissolved and the solution darkened. About 60 ml acetone were then distilled. The residue was allowed to cool to 50°, and most of the rest of the acetone was removed under vacuum with the temperature kept below 60°. Some solid appeared in the dark residue, and the resulting paste was poured into 500 ml stirred distilled water. A precipitate formed and was filtered from the dark solution, then washed on the filter with methanol (3 x 40 ml) and dried under vacuum to give 20 g (37%) off-white powder, m. p. 92-93°. Spectra (nmr, ir) were consistent with the assigned structure.

## SPECIFIC EXAMPLE V

When tetraethylene glycol diamine (JEFFAMINE® EDR-192) was the bismaleamic acid precursor, a solid bismaleimide could not be prepared. Instead, a dark, viscous liquid formed which was more soluble than the other two products and could not be induced to solidify. The proton nmr.spectrum of this material appeared to be that of a mixture containing a major amount (ca. 70% by nmr) of the desired bismaleimide along with smaller portions of unidentified impurities.

## SPECIFIC EXAMPLE VI

Preparation of the bismaleimide from diethylene glycol diamine was carried out identically and afforded a very light tan solid, m.p. 154.5-156.5°. Spectra (nmr, ir) were consistent with the assigned structure.

Heating of these bismaleimides alone at 180°-200° for 0.5-1.0 hours gave hard, transparent, somewhat brittle brown polymers with decomposition temperatures found by thermogravimetric analysis to be approximately 440° C (bisaminoethyl ether derivative) and 400° C (triethylene glycol diamine derivative.

## SPECIFIC EXAMPLE VII

Example VII demonstrates the preparation of the bismaleimidoaspartimide from the triethylene glycol bismaleimide. In a 250 ml 3-necked round-bottomed flask, under nitrogen, a solution of triethylene glycol diamine (1.68g, 11.3 mmol) in 10 ml chloroform is added dropwise to a solution of triethylene glycol bismaleimide (7.00g, 22.7 mmol) in 40 ml chloroform at 40°. The resulting orange solution is heated at reflux for 1.5 hours and then concentrated under vacuum to give immediately 8.1g of the prepolymer as a viscous orange liquid.

## SPECIFIC EXAMPLE VIII

Example VIII demonstrates the use of the bismaleimidoaspartimide in polymer preparation. A 17.5g sample of the prepolymer was heated at 60° under vacuum for 3 hours, then poured into a 5″ x 3.25″ mold which was placed into an oven at 125°. The oven was heated to 165° over a one hour period and then held at 165-175° for an additional two hours. The product polymer was obtained as a hard, red, fairly stiff plaque with a room temperature flexural modulus (ASTM D-790) of 460,000 psi.

Other examples include bismaleimides of 2,4-toluenediamine, 4,4′-diaminodiphenylmethane, 1,4-diaminobenzene and 1,3-diaminobenzene.

One aromatic bismaleimide which works well is 4,4′-bismaleimidodiphenylmethane (MDABMI), shown below:

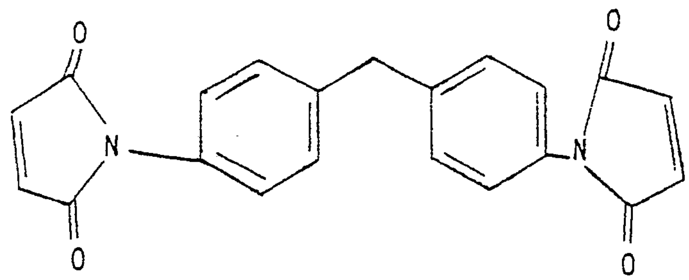

Examples for the Preparation of the Bismaleimide Derivatives of Higher Molecular Weight Polyoxyalkyleneamines

The novel bismaleimides are prepared by reacting low molecular weight oxyethylenediamines with higher molecular weight polyoxyalkyleneamines to form bis (maleimidoaspartimide) compounds. As mentioned the reaction is a Michael addition. The reaction works particularly well with propylene oxide capped polyoxyalkyleneamines. The low molecular weight bismaleimides react with the propylene oxide capped diamines in a 2/1 ration to give bis-Michael adducts.

In the method of the instant invention the bismaleimide is dissolved in chloroform and the polyoxyalkyleneamine is gradually added. The resulting solution is stirred at a temperature of from 20°C to 140°C with the preferred range being 40-80°C for a period of from 1 to 4 hours. The chloroform is then removed under vacuum with the temperature kept below 55°C. In most cases the product is a liquid at room temperature.

In the method of this invention, the pressure may range from 0.5 atm to 50 atm. The preferred pressure is atmospheric.

The Polyoxyalkyleneamines used for the preparation of derivatives with higher molecular weight.

Generally, polyoxyalkyleneamines which will work are those with a molecular weight of greater than 200.

One group of suitable amines are polyoxyalkylenediamines of the formula:

$NH_2CH(CH_3)CH_2\text{-}[OCH_2CH(CH_3)]_x\text{-}NH_2$

Compounds having the above formula include JEFFAMINE® D series diamines which are based on a polypropylene glycol (PPG) backbone and are available in a variety of molecular weights. They are low viscosity, light-colored liquids exhibiting low vapor pressure and high primary content. D-series amines include D-230, D-400 and D-2000 with approximate molecular weights of 230, 400 and 2000 respectively. Good results were obtained using D-400 as demonstrated in Specific EXAMPLE I.

Another group which can be used is water-soluble diamines based on a predominantly polyethylene glycol (PEG) backbone of the formula:

$NH_2CH(CH_3)CH_2\text{-}[OCH(CH_3)CH_2]_x\text{-}[OCH_2CH_2]_y\text{-}[OCH_2CH(CH_3)]_zNH_2$

Polyoxyethylenediamines having this formula include the JEFFAMINE®-ED series diamines. ED series include ED-600, ED-900 and ED-2000, having approximate molecular weights of 600, 900 and 2000 respectively.

Aromatic Bismaleimides

Where aromatic bismaleimide derivatives of polyoxyalkyleneamines are the desired product suitable aromatic bismaleimides can be selected from among those which are commercially available.

One suitable aromatic bismaleimide has the formula:

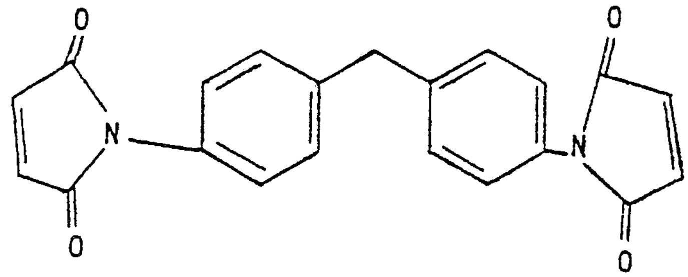

Preparation of the aromatic bismaleimide derivatives should be carried out under a temperature of from 45° C to 75° C. The preferred temperature is under approximately 125° C.

The products resulting from this method are used to make polymers by reacting them at a temperature of at least 150° C for about 30 minutes to 2 hours in the presence of either an additional amount of the higher molecular weight bismaleimide derivative or in the presence of a suitable copolymer.

The following examples are given in the way of illustration only and are not intended as limitations on the scope of the invention.

Examples I through IV demonstrate the preparation of bismaleimide derivatives of higher molecular weight polyoxyalkyleneamines.

In these preparations the diamine (neat or in chloroform solution) is added to a chloroform solution of the oxyethylene bismaleimide. The following illustrates the procedure for two different polyoxyalkyleneamines.

## SPECIFIC EXAMPLE I

The bismaleimide of bisaminoethyl ether (24.5g, 93 mmol) was dissolved in 175 ml chloroform at 55° C in a 500 ml 3-necked round bottom flask; the D-400 (18.6g, 46 mmol) was added over a 10 minute period. The resulting solution was heated at 55° C for an hour and then allowed to sit at room temperature overnight. The chloroform was removed under vacuum, with the temperature kept below 55° C, leaving a tan paste, 47g, 110% (chloroform not entirely removed. The nmr analysis of this material was as expected for the desired product.

## SPECIFIC EXAMPLE II

The bismaleimide of JEFFAMINE® EDR-148 (triethylene glycol diamine) 14.1g, 45.8 mmol) was dissolved in 100 ml chloroform in a 250 ml 3-necked round-bottomed flask. The ED-900 (20.0g, 22.2 mmol) was added in a steady stream, and the resulting orange solution was heated at 65° C for 2.5 hours. The chloroform was then removed under vacuum, with the temperature kept below 55° C, giving a viscous, rather turbid brown liquid, 33.1g, 97%. The proton nmr spectrum of this material was as expected for the desired product.

Use of these products in preparation of polymers, both alone and with other bismaleimides, is illustrated in the following examples:

## SPECIFIC EXAMPLE III

The product of Example II above (1.0g) was heated with the bismaleimide of JEFFAMINE® EDR-148 (1.0g) at 185° C for 45 min. The product was a clear, hard, dark orange polymer with a decomposition temperature of 380° C determined by tga in nitrogen.

## SPECIFIC EXAMPLE IV

The product from Example I above (2.6g) was heated at 180° C (after a brief initial heating to 215° C) for one hour. The product was a clear, flexible, orange polymer with a decomposition temperature of 270° C determined by tga in nitrogen.

Examples V through IX demonstrate the method of producing an aromatic bismaleimide derivative and the use of the products in homopolymerization and copolymerization. In a general preparation of the compounds of this invention, a chloroform solution of the polyoxyalkylene diamine is added to a chloroform solution of the aromatic bismaleimide. The resulting solution is optionally heated and then concentrated under vacuum to afford the bismaleimide product, which can be polymerized.

## SPECIFIC EXAMPLE V

Reaction of JEFFAMINE® D-400 with 4,4′-bismaleimidodiphenylmethane (MDABMI):

The MDABMI (8.52g, 23.8 mmol) was dissolved in 45 ml chloroform at 40° C in a 250 ml 3-necked round-bottomed flask under nitrogen. A solution of JEFFAMINE® D-400 (4.81g, 12.0 mmol) in 5 ml chloroform was added dropwise over a 5-10 minute period, resulting in a darkening of the solution. The chloroform was removed under vacuum, with the solution heated in a hot water bath, leaving 12.5g of glassy orange solid.

## SPECIFIC EXAMPLE VI

Reaction of JEFFAMINE® ED-900 with MDABMI:

The MDABMI (6.00g. 16.6 mmol) was dissolved in 40 ml chloroform at 45° C. A solution of ED-900 (7.52g, 8.35 mmol) in 5 ml chloroform was added dropwise over a 5-10 minute period; the resulting orange solution was stirred at 40-45° C for two hours, then concentrated under vacuum (heating with hot water bath) to 12.0g of a very viscous orange liquid.

## SPECIFIC EXAMPLE VII

Reaction of JEFFAMINE® EDR-148 (triethyleneglycol diamine) with MDABMI:

The MDABMI (8.00g, 22.3 mmol) was dissolved in 50 ml chloroform and heated to 40° C The EDR-148 (1.65g, 11.1 mmol) was dissolved in 8 ml chloroform and added dropwise over a 10-15 minute period. The resulting solution was stirred at 40° C for two hours, then filtered from a small amount of solid and concentrated under vacuum to a yellow glass that softened at approximately 90-105° C.

## SPECIFIC EXAMPLE VIII

Homopolymerization of JEFFAMINE® D-400/MDABMI adduct:

A 1.5g sample of the glass was heated to 200° C over a 45 min period and held at that temperature for 35 min. The product was a hard, opaque red polymer with a decomposition temperature not yet determined.

## SPECIFIC EXAMPLE IX

Copolymerization of JEFFAMINE® ED-600/MDABMI adduct with JEFFAMINE EDR-148/EDR-148 BMI adduct:

A mixture of 11.3g of ED-600 adduct and 3.38g of EDR-148/EDR-148 bismaleimide adduct was heated under vacuum at 65° C for two hours and then poured into a 7" x 3.25" x 0.125" mold. The mold was heated in an oven at 160-170° C for 2.75 hr, and the product polymer was obtained as a red, semitransparent plaque with some voids resulting from evolution of volatiles. No properties have been determined.

## SPECIFIC EXAMPLES X-XXII

Small samples of some of the polymers were prepared from these materials by heating the prepolymers in a rectangular mold in air at 170° C 185° C A brief description of their properties is given in the table below. Amine(s) refers to the amine reacted with the BMI source (Michael acceptor).

| Example | amine(s) | BMI source | properties |
|---|---|---|---|
| X | D-2000 | MDA | red, flexible, inelastic |
| XI | T-5000 | EDR-148 | orange, flexible, elastic |
| XII | D-400 | EDR-148 | red, flexible, inelastic |
| XIII | ED-900 | EDR-148 | brown, flexible, slightly elastic |
| XIV | T-403 | EDR-148 | red, somewhat flexible, inelastic |
| XV | D-400 | BAEE | red, flexible, inelastic |
| XVI | T-5000 | MDA/BAEE | clear, yellow, elastic |
| XVII | D-400/T-403 | EDR-148 | clear, red, flexible |
| XVIII | ED-2001 | EDR-148 | orange, translucent, rather flexible, inelastic |
| XIX | EDR-148/D-4000 | EDR-148 | brown, opaque, very flexible and elastic |
| XX | EDR-148/T-5000 | EDR-148 | brown, opaque, flexible and strong |
| XXI | ET-3000 | EDR-148 | transparent, flexible, elastic |
| XXII | D-230 | EDR-148 | red, translucent, somewhat flexible, inelastic |

Amines and BMI sources beginning with D-, T-, ED-, EDR-, or ET- refer to the JEFFAMINE amine products designated as shown. BAEE is bis(aminoethyl)ether and MDA is 4,4′-methylene dianiline. In Example XVI the molar ratio of MDA to BAEE is 1:1.

## Claims

1. A bismaleimide derivative characterised in that it has the formula A or B and that it is formed at a temperature sufficient to cause thermal reaction between heated components of the mixture of:
(a) at least one diamine of the formula
$H_2NCH_2CH_2$-(-$OCH_2CH_2$-$)_n$-$NH_2$
wherein n equals 2, 3 or 4 and
(b) maleic anhydride
wherein components (a) and (b) form a bismaleamic acid represented by the formula

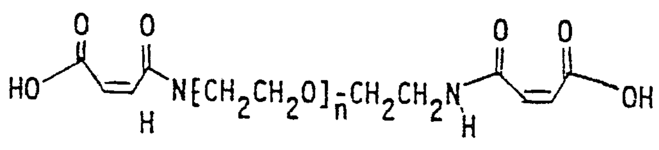

which is further reacted with acetic anhydride to form a bismaleimide of the formula A:

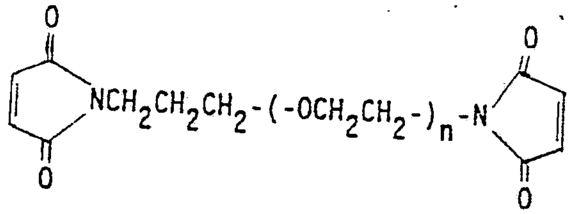

wherein n = 2, 3 or 4, which optionally can be further reacted with a polyoxyalkyleneamine with a molecular weight greater than about 200 resulting in a bismaleimide derivative of a higher molecular weight polyoxyalkyleneamine of formula B:

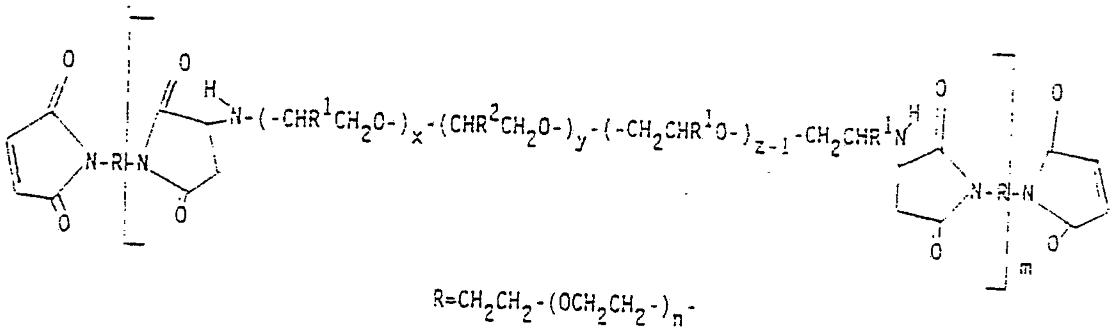

$R{=}CH_2CH_2\text{-}(OCH_2CH_2\text{-})_n\text{-}$

wherein $R^1$ and $R^2$ are independently alkyl or hydrogen, x, y and z depend on the amine used, n is 1, 2, or 3, and m is predominantly 1.

2. A derivative according to claim 1 characterised in that the diamine component is selected from diethylene glycol diamine, triethylene glycol diamine and tetraethylene glycol diamine, bisaminoethyl ether, JEFFAMINE® EDR-148 and JEFFAMINE® EDR-192.

3. A bismaleimide derivative according to claim 1 characterised in that it has the formula:

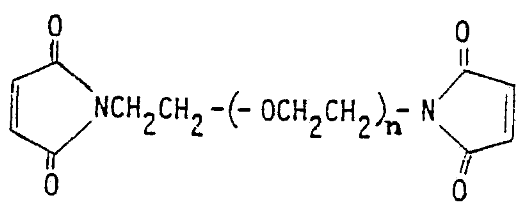

wherein n = 1, 2 or 3, with bisaminoethyl ether as the diamine reactant for n=1, with JEFFAMINE® EDR-148 as the diamine reactant for n=2, and with JEFFAMINE® EDR-192 as the diamine reactant for n=3.

4. A copolymer wherein one polymer comprises a bismaleimide compound of the formula:

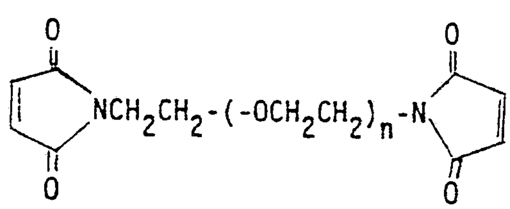

wherein n equals 2, 3 or 4 and wherein the copolymer comprises a compound selected from the group consisting of aliphatic or aromatic diamines and unsaturated compounds.

5. A method of preparing a bismaleimide derivative characterised in that it comprises reacting a polyoxyalkyleneamine in a chloroform solution with an aromatic bismaleimide in a chloroform solution, optionally heating the solution and subjecting the solution to a vacuum to recover the aromatic bismaleimide product.

6. A method according to claim 5 characterised in that the polyoxyalkyleneamine is of the formula
$H_2N\text{-}(\text{-}CHR^1CH_2O\text{-})_x\text{-}(\text{-}CHR^2CH_2O\text{-})_y\text{-}(\text{-}CH_2CHR^1O\text{-})_{z\text{-}1}\text{-}CH_2CHR^1NH_2$
where $R^1$ and $R^2$ are independently, alkyl or hydrogen, and x, y, and z depend on the amine used.

7. A method according to claim 5 characterised in that the polyoxyalkyleneamine is of the formula:
$NH_2CH(CH_3)CH_2\text{-}[OCH_2CH(CH_3)]_x\text{-}NH_2$,
$NH_2CH(CH_3)CH_2\text{-}[OCH(CH_3)CH_2]_x$
or
$H_2N\text{-}CH_2CH_2(OCH_2CH_2)_n\text{-}NH_2$
where n = 1 or 2.

8. A method according to any of claims 5 to 7 characterised in that the aromatic bismaleimide is 4,4′-bismaleimidodiphenylmethane.

9. A method according to any of claims 5 to 8 characterised in that the solution is heated at a temperature of from 50° C-125° C.

10. A method for preparing a liquid bismaleimidoaspartimide prepolymer from the solid bismaleimide of the formula:

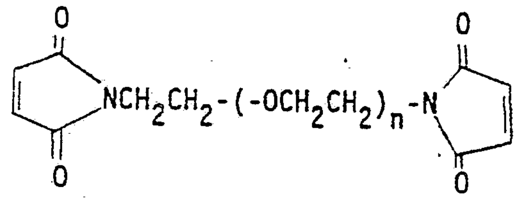

characterised in that it comprises reacting said solid bismaleimide with triethylene glycol diamine at a temperature of from 0° C to 125° C to form a liquid prepolymer suitable for polymerization.

11. A liquid bismaleimidoaspartimide prepolymer characterised in that it comprises 60 to 90 weight percent bismaleimide of the formula:

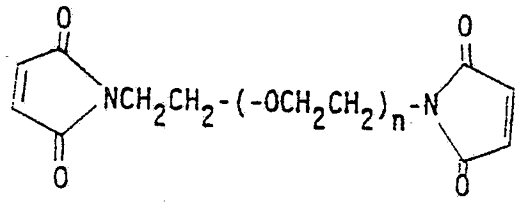

wherein n = 2, 3 or 4, and 10 to 40 weight percent triethylene glycol diamine.

12. A novel aromatic bismaleimide derivative characterised in that it has the formula:

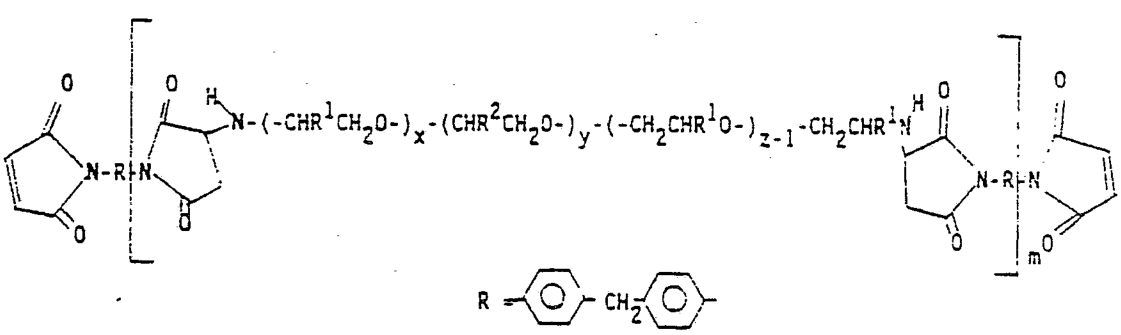

wherein $R^1$ and $R^2$ are independently alkyl or hydrogen, x, y, and z depend on the amine used, and m is predominantly 1.

13. Any homopolymer or copolymer characterised in that it is formed by using the bismaleimide of claim 12.